# EUROPEAN PATENT SPECIFICATION

(11) **EP 3 210 499 B1**
(45) Date of publication and mention of the grant of the patent: **15.08.2018**
(21) Application number: 17157117.7
(22) Date of filing: 21.02.2017
(51) Int. Cl.: A47C 7/74, A47C 1/14, A61N 5/06, A47C 7/72, A47C 21/00

(54) **STRUCTURE FOR A SUNBED, DECKCHAIR, TELESCOPIC TRIPOD OR TANNING AND REFRESHING PLATFORM**
STRUKTUR FÜR EINE GARTENLIEGE, EINES LIEGESTUHLS, EINES TELESKOPISCHEN STATIVS ODER BRÄUNUNGS- UND ERFRISCHUNGSPLATTFORM
STRUCTURE POUR LIT DE BRONZAGE, CHAISE LONGUE, TRIPODE TÉLESCOPIQUE OU PLATE-FORME DE BRONZAGE ET DE RAFRAÎCHISSEMENT

(30) Priority: 25.02.2016 IT UB20161074
(43) Date of publication of application: 30.08.2017
(73) Proprietor: Arredamenti Colombo Giovanni, 22063 Cantu' (CO) (IT)
(72) Inventor: COLOMBO, Giovanni, 22063 Cantu' (CO) (IT)
(74) Representative: Rastelli, Franco

(56) References cited:
- WO-A1-2015/127487
- DE-U1- 7 624 114
- US-A- 3 625 434
- US-A- 5 975 630
- US-B1- 8 123 290
- US-B1- 8 123 291
- Unknown: "The Solar Powered Entertainment Lounger - Hammacher Schlemmer", , 7 December 2015 (2015-12-07), XP055299001, Retrieved from the Internet: URL:http://web.archive.org/web/20151207134 103/http://www.hammacher.com/Product/Defau lt.aspx?sku=12425&promo=Outdoor-Living-Fur niture&catid=1082 [retrieved on 2016-08-31]

## Description

### BACKGROUND OF THE INVENTION

The present invention concerns a structure for a sunbed, deckchair, telescopic tripod or tanning and refreshing platform.

The habit of sunbathing, or even tanning with UV lamps, comfortably lying on sunbeds or in deckchairs, on the beach or in purposely designed solariums, is widespread.

For said purpose numerous types of sunbeds and deckchairs have been produced which, although they provide a good supporting function, cannot solve certain problems linked to exposure to the sun's rays or to UV radiations, consisting in the fact that the heat can become difficult to withstand and the user may have to refresh himself/herself before continuing the exposure.

Said need may be difficult to meet for various reasons.

For example, it may not be possible to wet the whole body for health reasons.

There may not be any showers or a beach nearby.

Furthermore, the tanning would have to be interrupted for a few minutes, and in the case of exposure to lamps in purposely-designed solariums, this time would be charged to the customer.

The difficulty or in any case the inconvenience of wetting the parts of the body exposed to the ultraviolet and/or the infrared radiations is reflected, furthermore, in a tan which takes longer to obtain.

In fact, the heat can become unbearable and consequently the person will discontinue the exposure.

Furthermore, dry skin hinders the tanning process compared to wet skin, since the drops of water on the skin produce a lens effect, which concentrates the incident radiations, increasing their effectiveness.

### SUMMARY OF THE INVENTION

The aim of the present invention is to provide a structure for a sunbed, deckchair, telescopic tripod or tanning and refreshing platform which is able to eliminate the above-mentioned drawbacks.

According to this aim, one object of the invention is to provide a structure of the type indicated which can be produced either as a newly built structure or by means of simple modifications of existing structures, also metallic.

A further object is to provide a structure of the type indicated which is inexpensive in production terms, dependable and easy to maintain.

A further object of the present invention is to provide a structure of the type indicated which does not have to be connected to a standard electricity supply network.

A further object of the present invention is to provide a structure of the type indicated which, when used for sunbathing, enables the user to adjust to the position of the sun which, as is known, varies throughout the day.

A further object of the present invention is to provide a structure of the type indicated which is compact and, if desired, foldable so that it can be transported in the boots of commonly used cars and/or vans.

A further object of the present invention is to provide a structure of the type indicated which, in addition to an effective refreshing effect, can also have toning and/or curative effects on the user's skin.

These and further objects which will become apparent below are achieved by a structure for a sunbed, deckchair, telescopic tripod or tanning and refreshing platform having the characteristics of appended claim 1.

### BRIEF DESCRIPTION OF THE DRAWINGS

Further characteristics and advantages of the present invention will become more apparent from the present detailed description of preferred embodiments thereof, illustrated only for descriptive and non-limiting purposes, in the accompanying drawings, in which:
FIG. 1 is a schematic perspective view of the modular nebulizer device which can be combined with the structure of the sunbed, telescopic tripod and/or similar of the present invention and which is the gist of the present invention;
FIG. 1A is a further perspective view of the modular nebulizer device of FIG. 1, with the tank cap removed from the tank;
FIG. 2 is a perspective view of a possible configuration of a sunbed with the modular device of FIG. 1 and FIG. 1A applied thereto;
FIG. 3 is a further perspective view of a further possible embodiment of the sunbed and/or refreshing bed of the present invention with the modular device of FIG. 1 and FIG. 1A applied thereto and with a tanning system, for example with UV lamps;
FIG. 4 shows a further possible configuration of the sunbed of the present invention which can be directed in a revolving manner to follow, for example, the position of the sun which, as is known, varies throughout the day; and
FIG. 5 shows a further application of the modular nebulizer device combined with a telescopic tripod.

### DESCRIPTION OF THE PREFERRED EMBODIMENTS

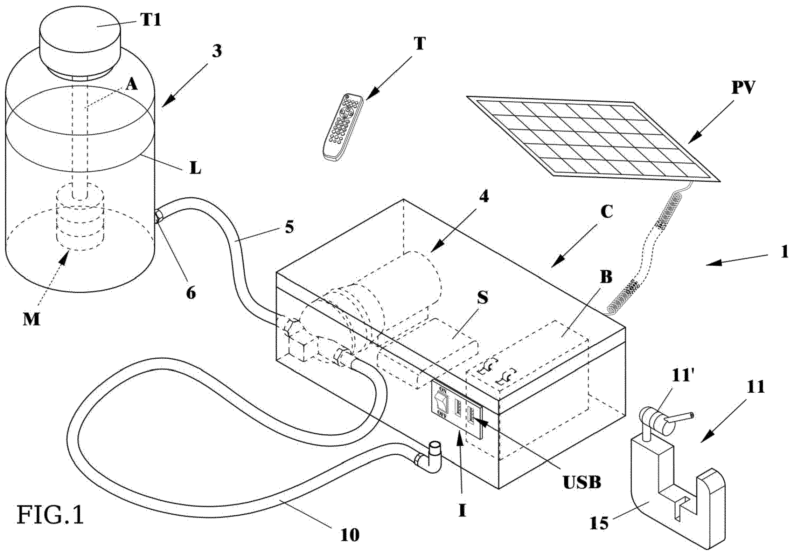

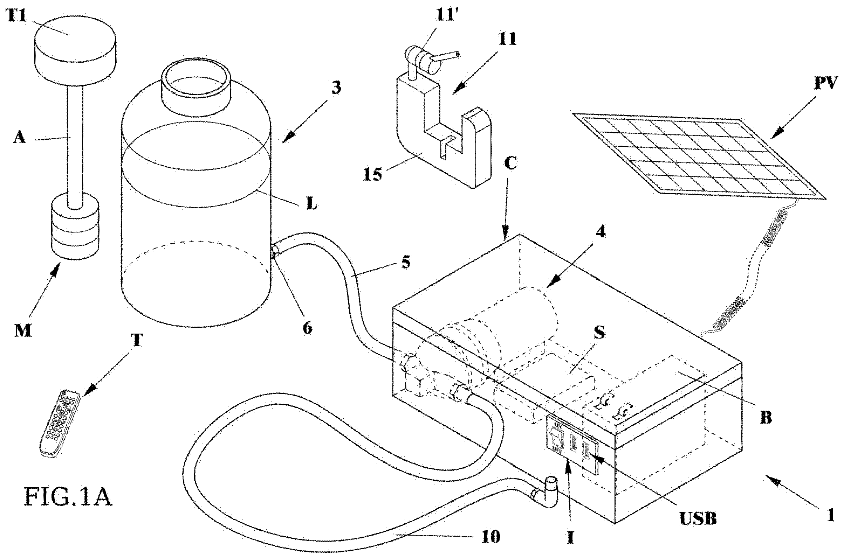

With specific reference to the cited figures, and in particular to FIGS. 1 and 1A, they show the nebulizer module which is the gift of the present invention, and is specifically designed to be applied to the cited sunbeds, deckchairs, telescopic tripods, sunbathing platforms and/or similar, also constituting a main aspect of the present invention.

In FIG. 1 the nebulizer module is generally indicated by the reference number 1.

It should be noted that the nebulizer module 1 has been designed as an independent unit which, in addition to the cited applications, can also be marketed for application in so-called "hot areas" to refresh said areas by its nebulization action, as will be described in greater detail below.

As can be noted, the nebulizer module 1 comprises as its main component parts a substantially cylindrical tank 3 with capacity of a few litres suitable for being filled with liquids in general, for example ordinary water, the level L of which is indicated; said tank has a connector 6 connected, by means of a hose 5, to the end of an electric pump generally indicated by the reference number 4, which can be controlled by a button unit I, for example of the ON/OFF type.

Of course, this control button unit I can also consist of a timed touch control.

Advantageously the electric pump 4 is contained in a dedicated container, generally indicated by the reference letter C, which also contains an electric stabilizer S and a battery B of accumulators and USB socket assembly.

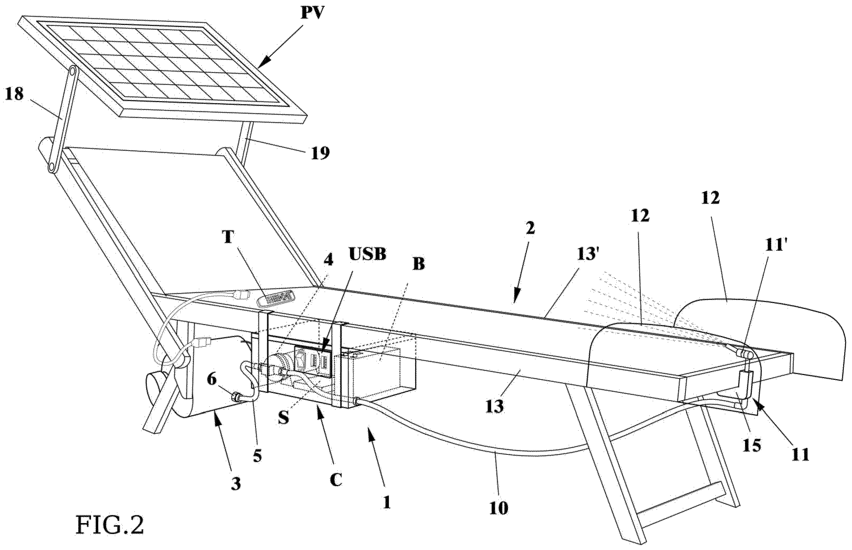

The pump 4 is connected downstream to a distribution hose 10 which conveys the liquid sent from the above-mentioned pump to at least one ejector or nebulizer assembly, generally indicated by the reference number 11, manually or electrically adjustable in the flow direction, and which is mechanically fixed to the base B' of the sunbed 2 and/or telescopic tripod rod (see FIG. 2).

The tank 3 containing liquid at level L can be applied either by means of a pair of belts running below it and fixed at one end in a removable manner to the bottom of the structure of the sunbed 2 and/or telescopic tripod CA (FIGS. 2 - 5), or by means of other support, for example a metal bracket, to which the device 1 will be applied.

Advantageously, a rod A is integral with the cap T1 of the tank 3; at the bottom of the rod a magnet unit M designed to magnetize the water can be applied in order to give the water, as is known in the art, particular beneficial properties for the skin and to complete the nebulizer module or several photovoltaic panels PV.

Naturally, fragrances and/or aromatic substances can be added to the tank, so that the aromatic water delivered by the nozzle 11' of the nebulizer 11 will also advantageously have, in addition to the desired refreshing properties, natural toning and detergent properties on the user's skin.

Advantageously the box-shaped container C, for example metallic, or "case" containing at least the pump 4, the stabilizer S and the battery B, can be easily applied to the side members 13 of the sunbed for example by means of quick couplings.

Also the nebulizer assembly 11 with its nozzle 11' can be advantageously mounted on a bracket 15, easily applicable to an element of the sunbed and in a desired position, thanks to the presence of the distribution hose 10.

Naturally, where necessary, a plurality of nebulizers 11, 11' can also be applied, and not only one, as in the embodiment example described.

In this case a plurality of pumps and/or switches and/or timed detectors and sensors, as well as distribution hoses, can be provided so that sets of ejector nozzles 11' can be selected to spray a certain area.

In all the embodiments, wind barriers can also be advantageously provided (FIGS. 2; 12), preferably made of transparent Plexiglas, connected for example to the structure of the element to protect the nebulizers.

Said wind barriers are particularly useful for an outdoor use of the device in question, in order to stabilize, in windy conditions, the direction of the jet coming out of the ejector nozzles 11'.

With reference to FIG. 2, a photovoltaic panel assembly PV has been advantageously connected to the sunbed in a tilting manner and can be folded onto respective hinged arms 18 and 19; said photovoltaic panel assembly being designed to transform the solar energy into electrical energy so that the sunbed, and in particular all the components in the nebulizer module 1, will be supplied by the photovoltaic panel PV and therefore without requiring any connection to the electricity or water network.

The photovoltaic panel or, if necessary, the photovoltaic panels PV form an integral part of the nebulizer module of the invention.

According to a further aspect of the invention, below the side member 13 of the sunbed, a USB socket assembly is provided electrically powered by the cited photovoltaic panel PV and controlled for example by an ON/OFF switch to recharge electronic devices in general, for example a conventional mobile phone.

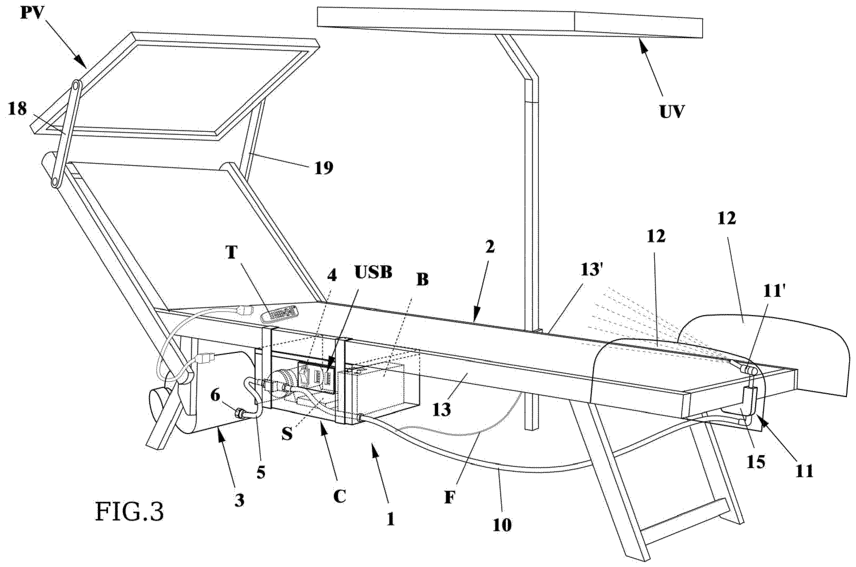

FIG. 3 shows, in addition to the solar panel, a UV lamp assembly associated with the side member 13' of the sunbed 2; said UV lamp assembly being powered via a supply wire F, for example from the battery container in the box C, in turn charged, via the stabilizer S, by the photovoltaic panel PV.

Naturally, in this regard, it should be appreciated that instead of one single photovoltaic panel, depending on the required utilization power, further photovoltaic panels can be provided appropriately positioned and connected.

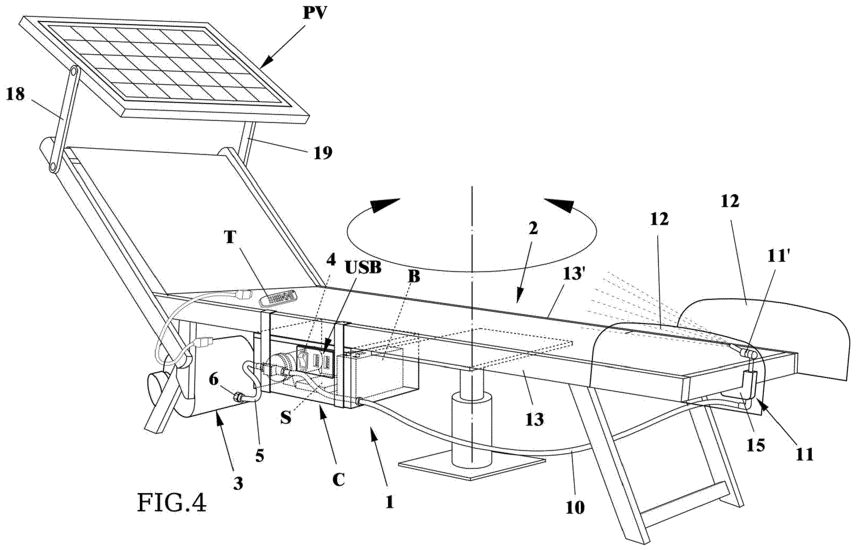

FIG. 4 shows a further possible embodiment of the structure of the sunbed and/or similar, according to the present invention, which can be easily oriented, even automatically, by the user, for example to follow the movement of the sun, the rays of which, as is known, change their inclination and therefore their tanning effectiveness throughout the day. As can be noted, the orientation, i.e. the rotation of the sunbed 2, is simply obtained by means of a cylinder-piston assembly C1, the control means of which are not shown for the sake of simplicity.

The orientation of the sunbed 2 or tripod CA can be obtained by means of any other known device, also directly associated with the sunbed 2.

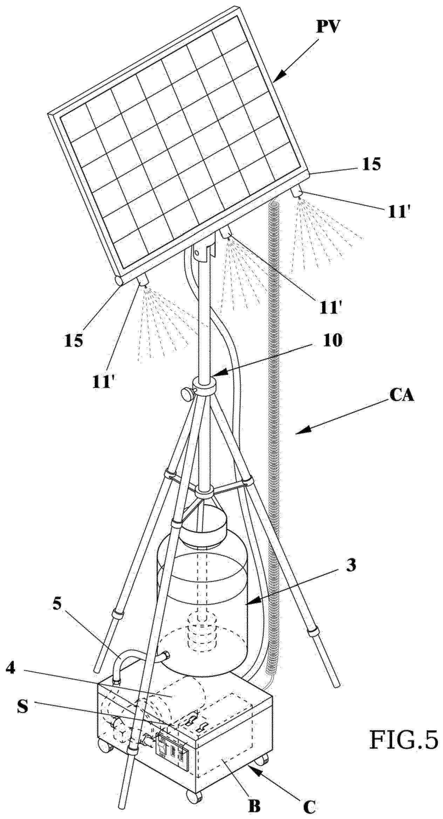

In FIG. 5 the nebulizer module of the invention, i.e. its basic components like the case C containing the pump and the battery, as well as the tank 3, are mounted on an upright or tripod structure CA, together with at least one nebulizer nozzle 11', actually in this case three nozzles 11', thus defining a nebulizer structure which can also include a greater number of nebulizer nozzles 11', able to refresh any hot area as desired, with the beneficial effects of the magnetically treated nebulized water and/or with the addition of fragrances or substances which are beneficial for the skin.

A photovoltaic panel PV is also mounted on the tripod CA, said photovoltaic panel being electrically connected by means of a connector cable CC to the box C to supply electricity to the components, already described, associated with it.

Also said tripod refreshing structure CA constitutes a particular aspect of the invention since it directly uses the refreshing module of the invention, therefore it is independent of both any electrical and water networks that may be present in the area to be refreshed.

Naturally, the shapes and dimensions of the various component elements of the structure of the sunbed and/or similar according to the invention, and of the liquid ejector device according to the invention, particularly suitable for application on the cited sunbeds, deckchairs, telescopic tripod and sunbathing platforms, can vary according to requirements, while remaining within the scope of the inventive concept illustrated above.

The operation of the device, according to the invention, can be easily deduced from the present description.

After filling the tank 3 with water which, as said, can be magnetized by the magnet M with the optional addition of any fragrances and/or curative substances for the skin, a user lying on the sunbed 2 will operate the switch of the pump 4 as he/she likes, thus determining the outflow of nebulized water from the nebulizer nozzle 11'.

It has been found in practice that the invention fully achieves the intended aim and objects..

In fact, a structure for a tanning and refreshing sunbed is provided which includes a modular nebulization unit, with electric pump directly powered by solar panels; said modular unit can be used separately in any desired application, in any "hot" area where users wish to benefit from both refreshing and toning, for example it can be easily and quickly mounted on the cited tripod CA.

Although the invention has been described and illustrated with specific reference to currently preferred embodiments thereof, it should be apparent that said embodiments are only provided by way of examples and are susceptible to variations and modifications all falling within the scope of the appended claims.

For example, the tripod CA can be replaced by a simple telescopic rod adjustable to different height levels.

Furthermore, the liquid container can be also inserted in the box C, thus providing a compact folding unit also usable in a domestic context for spraying plants.

## Claims

1. A structure for a sunbed, deckchair, telescopic tripod or sun or UV ray tanning and refreshing platform for hot areas in general, to said structure being removably associated a nebulizer module (1) comprising liquid tank means (3) operatively connected to electric pump means (4) supplied with said liquid, and at least one nebulized liquid ejector nozzle (11'); said nebulizer module (1) comprising a box-shaped container body containing at least, operatively electrically interconnected, said electric pump means (4), electrical energy accumulator means (B), electrical energy stabiliser means (S) and USB socket assembly, electrical energy being produced by at least one photovoltaic panel (PV) removably associated in an adjustable orientation with said structure, **characterized in that** said tank means (3) comprise a removable cap (T1) to which is operatively connected a permanent magnet (M) adapted to magnetize the liquid, specifically water, contained in said tank means (3).

2. A structure, according to claim 1, **characterized in that** said structure is removably associated with at least one UV tanning lamp.

3. A structure, according to claim 1, **characterized in that** said tank is connected to said pump means by means of a first hose, and said pump means are connected to said at least one ejector nozzle by means of a second hose, said ejector nozzle being protected by a respective wind barrier.

4. A structure, according to any one of the preceding claims, **characterized in that** said at least one photovoltaic panel and/or said at least one UV lamp are controlled by ON/OFF switch means or by touch switch means or by timer means.

5. A structure, according to any one of the preceding claims, **characterized in that** said structure is designed to be oriented so as to follow the sun position changes.

6. A structure, according to any one of the preceding claims, **characterized in that** it comprises connector means, preferably of the USB type, to connect electronic devices such as mobile phones and/or similar to the electrical energy supplied by said at least one solar panel.

7. A structure, according to any one of the preceding claims, **characterized in that** said structure consists of wooden material and/or metallic material, preferably aluminium.

8. A sun or UV ray tanning and/or refreshing system including at least a structure for sunbed, deckchair and/or telescopic tripod or platform, according to any one of the preceding claims.

## Patentansprüche

1. Struktur für eine Gartenliege, einen Liegestuhl, ein teleskopisches Stativ oder eine Sonnen- oder UV-Strahlen-Bräunungs- und Erfrischungsplattfform für allgemein heiße Gebiete, wobei ein Zerstäubermodul (1), das Flüssigkeitstankmittel (3), die mit elektrischen Pumpmitteln (4) wirkverbunden sind, denen die Flüssigkeit zugeführt wird, und mindestens eine Ausstoßdüse (11') für zerstäubte Flüssigkeit umfasst, lösbar mit der Struktur verbunden ist;
wobei das Zerstäubermodul (1) einen kastenförmigen Behälterkörper umfasst, der, betriebsfähig elektrisch miteinander verbunden, mindestens die elektrischen Pumpmittel (4), Elektroenergie-Speichermittel (B), Elektroeonergie-Stabilisatormittel (S) und USB-Buchsenanordnung umfasst, wobei elektrische Energie durch mindestens ein Photovoltaikmodul (PV), das lösbar in einer verstellbaren Ausrichtung mit der Struktur verbunden ist, erzeugt wird, **dadurch gekennzeichnet, dass** die Tankmittel (3) eine lösbare Kappe (T1) umfassen, mit der ein Permanentmagnet (M) wirkverbunden ist, der angepasst ist, die Flüssigkeit, insbesondere Wasser, die in den Tankmitteln (3) enthalten ist, zu magnetisieren.

2. Struktur nach Anspruch 1, **dadurch gekennzeichnet, dass** die Struktur lösbar mit mindestens einer UV-Bräunungslampe verbunden ist.

3. Struktur nach Anspruch 1, **dadurch gekennzeichnet, dass** der Tank mittels eines ersten Schlauches mit den Pumpmitteln verbunden ist und die Pumpmittel mittels eines zweiten Schlauches mit der mindestens einen Ausstoßdüse verbunden sind, wobei die Ausstoßdüse durch eine entsprechende Windschutzvorrichtung geschützt ist.

4. Struktur nach einem der vorhergehenden Ansprüche, **dadurch gekennzeichnet, dass** das mindestens eine Photovoltaikmodul und/oder die mindestens eine UV-Lampe durch EIN/AUS-Schaltmittel oder durch Berührungsschaltmittel oder durch Zeitschaltmittel gesteuert werden.

5. Struktur nach einem der vorhergehenden Ansprüche, **dadurch gekennzeichnet, dass** die Struktur ausrichtbar gestaltet ist, um den Sonnenstandsänderungen zu folgen.

6. Struktur nach einem der vorhergehenden Ansprüche, **dadurch gekennzeichnet, dass** sie Anschlussmittel, vorzugsweise vom USB-Typ, umfasst, um elektronische Geräte, wie beispielsweise Mobiltelefone und/oder ähnliches, an die elektrische Energie, die durch das mindestens eine Solarmodul bereitgestellt wird, anzuschließen.

7. Struktur nach einem der vorhergehenden Ansprüche, **dadurch gekennzeichnet, dass** die Struktur aus Holzmaterial und/oder Metallmaterial, vorzugsweise Aluminium, besteht.

8. Sonnen- oder UV-Strahlen-Bräunungs- und/oder Erfrischungssystem, das mindestens eine Struktur für Gartenliege, Liegestuhl und/oder teleskopisches Stativ oder Plattform nach einem der vorhergehenden Ansprüche umfasst.

## Revendications

1. Structure pour transat, chaise longue, tripode télescopique ou plate-forme de bronzage au soleil ou aux UV et de rafraîchissement pour régions chaudes en général, étant associés de manière amovible à ladite structure un module de nébulisation comprenant un moyen réservoir de liquide connecté au niveau opérationnel à un moyen de pompage électrique alimenté en dit liquide, et au moins une buse d'éjection (11') de liquide nébulisé, ledit module de nébulisation (1) comprenant un corps en forme de boîte contenant au moins le moyen de pompage électrique (4) interconnecté électriquement au niveau opérationnel, un moyen accumulateur d'énergie électrique (B), un moyen stabilisateur d'énergie électrique (S) et un ensemble de prise USB, de l'énergie étant produite par au moins d'un panneau photovoltaïque (PV) associé de manière amovible en orientation ajustable à ladite structure, **caractérisée en ce que** ledit moyen réservoir (3) comprend un bouchon amovible (T1) auquel est connecté au niveau opérationnel, un aimant permanent (M) apte à magnétiser le liquide, spécifiquement de l'eau, contenu dans ledit moyen réservoir (3).

2. Structure selon la revendication 1, **caractérisée en ce que** ladite structure est associée de manière amovible à au moins une lampe de bronzage UV.

3. Structure selon la revendication 1, **caractérisée en ce que** ledit réservoir est connecté audit moyens de pompage au moyen d'un premier tuyau, et lesdits moyen de pompages sont connectées à ladite buse d'éjection au moyen d'un second tuyau, ladite buse d'éjection étant protégée par une barrière pare-vent respective.

4. Structure selon l'une quelconque des revendications précédentes, **caractérisée en ce que** ledit panneau photovoltaïque et/ou ladite au moins une lampe de bronzage sont contrôlés par un moyen de commutation MARCHE/ARRET ou par un moyen de commutation tactile ou par un moyen de temporisation.

5. Structure selon l'une quelconque des revendications précédentes, **caractérisée en ce que** ladite structure est conçue pour être orientée de manière à suivre les changements de position du soleil.

6. Structure selon l'une quelconque des revendications précédentes, **caractérisée en ce qu'**elle comprend un moyen de connexion, de préférence de type USB, pour connecter des dispositifs électroniques tels que des téléphones mobiles et/ou similaires à l'énergie électrique fournie par ledit panneau solaire.

7. Structure selon l'une quelconque des revendications précédentes, **caractérisée en ce que** ladite structure est composée de matériau à base de bois et/ou de matériau métallique, de préférence d'aluminium.

8. Système de brossage au soleil ou aux rayons UV et/ou de rafraîchissement comprenant au moins une structure pour transat, chaise longue, et/ou tripode ou plate-forme télescopique selon l'une quelconque des revendications précédentes.
